(19) Europäisches Patentamt
European Patent Office
Office européen des brevets

(11) EP 4 650 987 A1

(12) EUROPEAN PATENT APPLICATION

(43) Date of publication:
19.11.2025 Bulletin 2025/47

(51) International Patent Classification (IPC):
G06F 16/906 (2019.01)    G06N 20/00 (2019.01)

(21) Application number: 24386053.3

(52) Cooperative Patent Classification (CPC):
G06F 16/906; G06N 20/00

(22) Date of filing: 13.05.2024

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Fujitsu Limited
Kawasaki-shi, Kanagawa 211-8588 (JP)

(72) Inventors:
• KALOUSIOS, Chrysostomos
Slough SL1 2BE (GB)
• INAKOSHI, Hiroya
Kawasaki-shi, Kanagawa 211-8588 (JP)

(74) Representative: Haseltine Lake Kempner LLP
Cheapside House
138 Cheapside
London EC2V 6BJ (GB)

Remarks:
Amended claims in accordance with Rule 137(2)
EPC.

(54) DOWNSAMPLING

(57) A method comprising: categorizing each datapoint in a training dataset into primary subsets based on first and second attributes; selecting a specific value of the first attribute and dividing each of the primary subsets corresponding to the selected value into a plurality of auxiliary subsets; for each of the primary subsets corresponding to the selected value, downsampling the plurality of auxiliary subsets with respect to the other primary subsets, respectively, to generate a plurality of downsampled auxiliary subsets, wherein the downsampling comprises: for each datapoint in the auxiliary subset concerned, computing an average distance to the k furthest datapoints of the primary subset concerned in respect of the plurality of attributes other than the at least first and second attributes; and removing n datapoints of the auxiliary subset concerned having the largest computed average distance to generate the downsampled auxiliary subset concerned, where k and n are positive integers.

| S10 | Categorize training dataset into plurality of primary subsets |
| S20 | Select a value of first attribute |
| S30 | Divide each primary subset corresponding to selected value into auxiliary subsets |
| S40 | For each primary subset corresponding to the selected value, downsample the auxiliary subsets with respect to the other primary subsets, respectively, to generate downsampled auxiliary subsets |
| S50 | Generate downsampled training dataset |

FIG. 12

Processed by Luminess, 75001 PARIS (FR)

EP 4 650 987 A1

**Description**

The present invention is related to downsampling, and in particular to a computer-implemented method, a computer program, and an information programming apparatus.

Machine learning (ML) models have proven useful for predicting values of attributes of data based on the values in respect of other attributes of a data instance. For example, classifiers are useful for predicting a class of a data instance based on values of the data instance in respect of other attributes.

The accuracy and fairness of an ML model depends at least in part on the training data used in training the ML model. Training data may be unbalanced in respect of a class or another attribute. For example, training data may be unbalanced in respect of a so-called protected attribute, upon which the ML model is not configured to base its prediction. The more imbalance in a training dataset, the more inaccurate or unfair (e.g. in respect of a particular group) the resulting ML model may be.

Downsampling is useful in some situations to address imbalance in training data for training an ML model. However, conventional downsampling methods are not optimal and may lead to other problems in the downsampled training dataset.

In light of the above, a downsampling method is desired. Such a method may for example be applied in downsampling a training dataset for training an ML model.

The present invention is defined by the independent claims, to which reference should now be made. Specific embodiments are defined in the dependent claims.

According to an embodiment of a first aspect there is disclosed herein a computer-implemented method comprising: categorizing each datapoint in a training dataset into one of a plurality of primary subsets based on values of the datapoint in respect of (at least) first and second attributes (so that each primary subset corresponds to specific values (in respect) of (at least) the first and second attributes) (so that for each primary subset the datapoints in the subset share the same value for the first attribute and the same value for the second attribute), (wherein each datapoint is defined by (values in respect of) a plurality of attributes including the at least first and second attributes); selecting a specific value in respect of the first attribute and dividing each of the primary subsets corresponding to the selected value (in respect) of the first attribute into a plurality of auxiliary subsets; for each of the primary subsets corresponding to the selected value (in respect) of the first attribute, downsampling the plurality of auxiliary subsets with respect to the other primary subsets, respectively, to generate a plurality of downsampled auxiliary subsets; and generating a downsampled training dataset for training a machine learning, ML, model to predict (a value of) the first attribute, the downsampled training dataset comprising the datapoints of the downsampled auxiliary subsets and the datapoints of the primary subsets other than the primary subsets corresponding to the selected value (in respect) of the first attribute, wherein the downsampling comprises: for each datapoint in the auxiliary subset concerned, computing an average distance to the k furthest datapoints of the primary subset concerned (the distance) in respect of the plurality of attributes other than the at least first and second attributes; and removing (from the auxiliary subset concerned) n datapoints of the auxiliary subset concerned having the largest computed average distance (to their respective k furthest datapoints of the primary subset concerned) to generate the downsampled auxiliary subset concerned, where k and n are positive integers.

Reference will now be made, by way of example, to the accompanying drawings, in which:

Figure 1 is a graph illustrating a dataset;
Figure 2 is a graph illustrating a dataset;
Figure 3 is a diagram illustrating a dataset;
Figure 4 is a diagram illustrating a method;
Figure 5 is a diagram illustrating a method;
Figure 6 is a diagram illustrating a method;
Figure 7 is a graph illustrating a dataset;
Figure 8 shows graphs illustrating a dataset;
Figure 9 shows graphs illustrating a dataset;
Figure 10 shows graphs illustrating a dataset;
Figure 11 is a diagram illustrating a method;
Figure 12 is a diagram illustrating a method;
Figure 13 is a diagram illustrating a method;
Figure 14 shows graphs illustrating results; and
Figure 15 is a diagram illustrating an apparatus.

The following definitions may be used in the description but are not exhaustive.

Binary classification: a classification problem in which observations are categorized into one of two classes (for example True or False, 0 or 1, yes or no).

Supervised learning: a category of machine learning that uses labelled datasets to predict outcomes and recognize patterns.

Attribute: A training dataset comprises data instances each defined by a number of attributes. That is, a given data instance is defined by values in respect of the attributes. Attributes may be referred to as features or predictors or variables or properties.

Protected attribute: Features of a dataset that may not be used as the basis for decisions e.g. by an ML model. Protected attributes can be chosen because of legal requirements, moral values, etc. Some common protected attributes include age, gender, nationality, race, age, etc.

Privileged group: Groups that have historically been more likely to receive favorable labels in a machine learning classification task.

Unprivileged group: A group that is not privileged, i.e. a group other than the privileged group. The groups when a dataset is divided according to a protected attribute may correspond to privileged and unprivileged groups.

Imbalanced or unbalanced dataset: A dataset with skewed class and/or group proportions.

Degree of Imbalance: A notion that characterizes the amount of imbalance of a dataset. For example: mild imbalance (minority class comprises 20-40% of the dataset), moderate imbalance (minority class comprises 1-20% of the dataset), and extreme imbalance (minority class comprises <1% of the dataset).

Minority class: A class that makes up a small proportion of the dataset.

Majority class: A class that makes up a large proportion of the dataset.

Downsampling: Also known as undersampling, is the process of using a smaller set of a given dataset, for example to be used to train an ML model.

Oversampling: A method that duplicates or creates new synthetic examples of a given dataset.

Statistical parity difference: A metric that evaluates the fairness of a system. It is based on a principle that privileged and unprivileged groups should receive an equal proportion of positive labels.

Equal Opportunity difference: A fairness metric that evaluates the difference of True Positive Rates for privileged and unprivileged groups.

Average odds difference: A fairness metric that expresses the average of difference in False Positive Rate and True Positive Rate for privileged and unprivileged groups.

True Positive Rate: A metric to evaluate performance of a binary classification model. It is defined as the proportion of actual positive cases that were correctly identified by the model.

False Positive Rate: A metric to evaluate performance of a binary classification model. It is defined as the proportion of positive cases that were incorrectly identified as positive by the model.

k-Nearest Neighbours: A machine learning technique used for classification and regression tasks.

As an example illustrating imbalance in training data, a binary classification problem may be considered with two classes, C1 and C2, and one protected attribute that splits the dataset into two groups, G1 and G2 , (privileged and unprivileged). For simplicity only one protected attribute is considered here - generalization to more than one protected group will become apparent. Two notions of imbalance may be considered:

1. Class imbalance

2. Group imbalance

In this case the protected attribute is gender and the groups are Men and Women. It can be seen from the table below giving the number of data instances/records for each group/class combination that there are more False records than True (class imbalance) and there are more records for men than women (group imbalance).

|  | True | False |
|---|---|---|
| Women | 1152 | 8698 |
| Men | 6058 | 13188 |

An ML classifier learns more from the majority class present in training data which may affect accuracy and fairness.

Class imbalance may be considered mainly to affect accuracy and group imbalance may be considered mainly to affect fairness.

As an example, a training dataset for training an ML model (classifier) to predict the presence of absence of diabetes is considered, that is, a medical cohort analysis of diabetes is considered. The training dataset comprises data instances corresponding to human subjects and defined by values in respect of a plurality of attributes, for example any of pregnancy, glucose levels, blood pressure, skin thickness, insulin levels, BMI, diabetes pedigree function, age, and outcome.

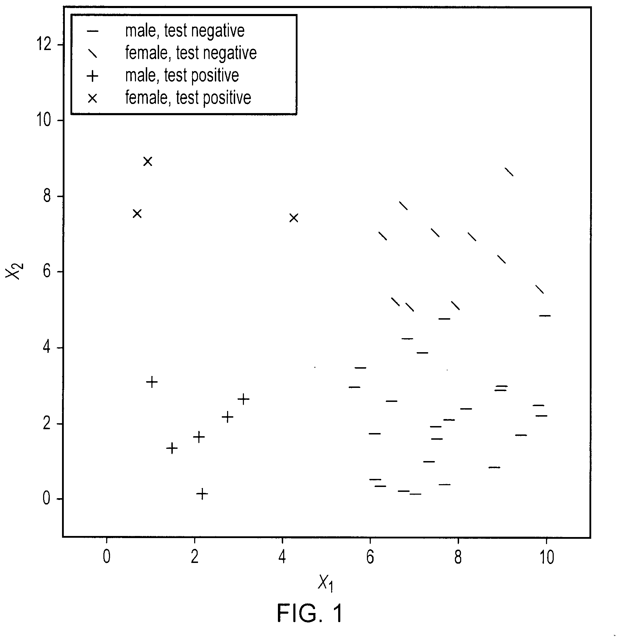

Figure 1 illustrates the datapoints of the training dataset across two attributes labeled $X_1$ and $X_2$ (which may for example be glucose level and BMI, or any other such attribute). Each datapoint corresponds to a data instance, i.e. to a human subject. The datapoints are categorized according to their group (male or female) and class (diabetes test positive or diabetes test negative). Most of the records are from male and are test negative. When training an ML model to predict the class (presence of diabetes), the resulting trained model can be inaccurate because it is trained by using dataset with fewer female records compared to male (and fewer positive recorded compared to negative).

A way to address an imbalanced classification problem is to change the composition of the training dataset. Such a technique is referred to as sampling. Sampling is only performed on the training dataset and not on the validation dataset.

Some conventional sampling methods include

- ∘ Random data sampling
- ∘ Oversampling methods
- ∘ Downsampling methods
- ∘ Combination of Oversampling and Downsampling

Conventional downsampling methods do not tend to consider fairness. In fact, after downsampling, fairness often deteriorates.

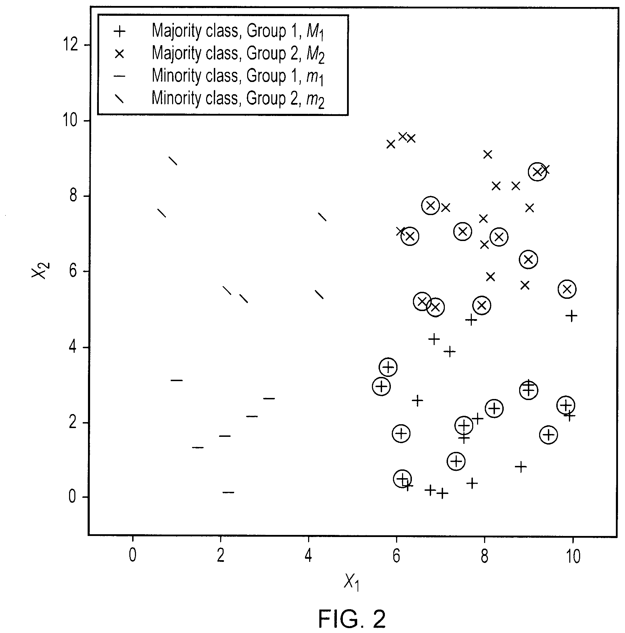

A comparative method will be described with reference to Figure 2. Figure 2 illustrates datapoints of a dataset according to their values in respect of attributes $X_1$ and $X_2$. The datapoints are categorized and labelled according to their group and class. The majority class is labelled with a plus - this is the class that is most common in the dataset. The categorization leads to the subsets of points M1, M2, m1 and m2 as shown in Figure 2.

In the comparative method, the subsets corresponding to the majority class are randomly downsampled. In random downsampling, points are randomly selected to be removed from the dataset (or are randomly selected to be included - and the remaining points are removed). For example, as shown in Figure 2 the result of random downsampling is that the circled points are removed from the dataset.

The benefit for fairness comes from balancing the size of different groups. However the above random downsampling approach may delete datapoints close to the boundaries between the subsets in the attribute space of $X_1$ and $X_2$. This may affect the prediction performance of an ML model trained using the downsampled dataset and may lead to underfitting.

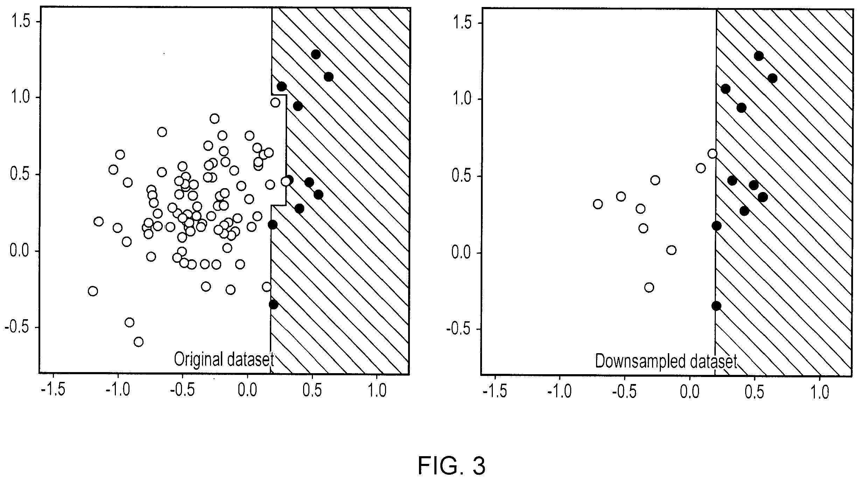

Figure 3 illustrates a representative example to demonstrate underfitting. On the lefthand-side (LHS) is a training dataset before downsampling and on the right-hand-side (RHS) is a training dataset after random downsampling. A classifier (DecisionTreeClassifier (https://scikit-learn.org/stable/modules/generated/sklearn.tree.DecisionTreeClassi fier.html, https://en.wikipedia.org/wiki/Decision_tree_learning)) learned from the original training dataset and the resulting decision boundary is shown on the LHS graph. The same classifier learned from the downsampled training dataset and the resulting decision boundary is shown on the RHS graph. After random downsampling the decision boundary has simplified to a straight line, thus leading to accuracy (and fairness) loss. The phenomenon is the opposite of overfitting, a possible negative effect of Oversampling.

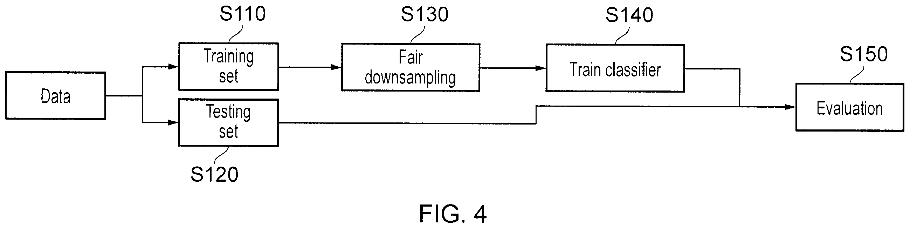

Figure 4 is a diagram illustrating an overview of a method for downsampling a training dataset and training an ML model (classifier). In steps S110 and S120, data is split into a training dataset and a testing dataset. In step S130 downsampling is performed on the training dataset. In step S140 the classifier is trained using the downsampled training dataset, and in step S150 the trained classifier is evaluated using the testing dataset (which has not been downsampled). Downsampling approaches disclosed herein may be implemented in the step S130. Methods disclosed herein include downsampling approaches and overall methods corresponding to the Figure 4 overview including such downsampling approaches in the step S130.

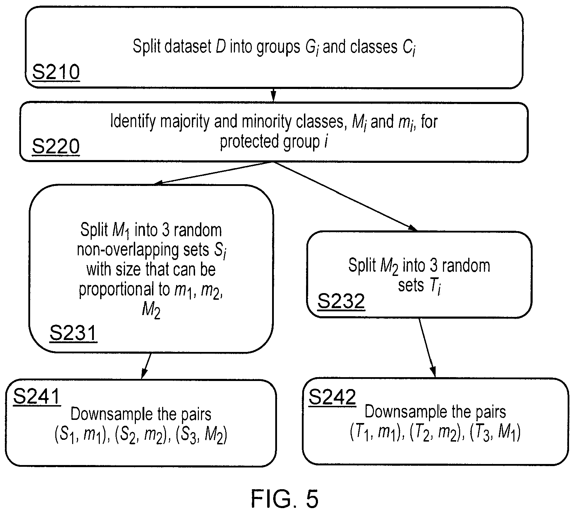

Figure 5 is a diagram illustrating a method. The method comprises steps S210, S220, S231, S232, S241, and S242.

The problem setup to which the method is applied is summarized as follows:

- Input: Observation/original dataset $D = \{X_i, Y_i, G_i\}$, $i = 1$ to n, where X refers to the features, i.e. the attribute values of the records, Y refers to the class of the records, G refers to the group of the records.

- Output: Downsampled dataset

- Evaluation metrics: Any of statistical parity difference, equal opportunity difference, equalized odds difference, balanced accuracy

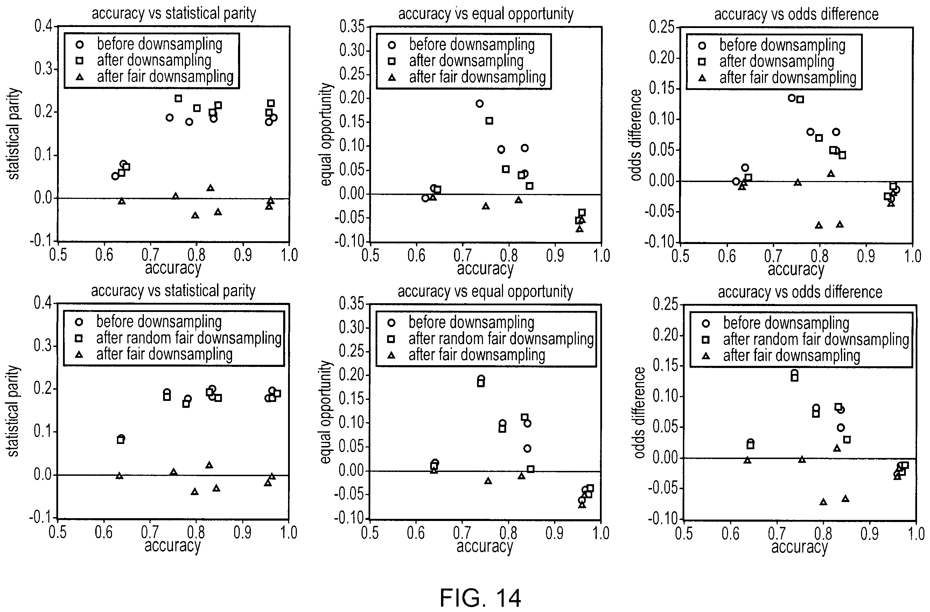

- Evaluation classifiers (used in evaluation as described below with respect to Figure 14): Any of LogisticRegression, SVC, RandomForestClassifier, KNeighborsClassifier, XGBClassifier, GaussianNB, GradientBoostingClassifier, DecisionTreeClassifier.

As an example, the Figure 5 method may be considered as relating to medical cohort analysis of diabetes using a distance-based fair downsampling approach. That is, in the example the input data is medical data including records corresponding to human subjects including values for attributes which may include, for example, any of pregnancy, glucose levels, blood pressure, skin thickness, insulin levels, BMI, diabetes pedigree function, age, and outcome. In this case, the class is whether or not the human subject has tested positive for diabetes and the group is either of male and female. As indicated above, the class is an attribute which the downsampled training dataset will be used to train an ML model to predict.

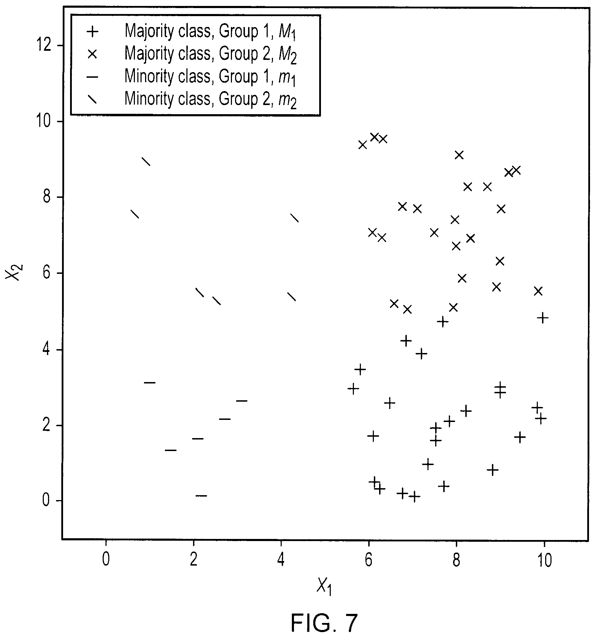

Step S210 comprises splitting the dataset D into groups Gi and classes Ci. This comprises splitting the dataset into classes (positive test for diabetes and negative test for diabetes) and then splitting again into groups (male and female) or vice versa. The result in this case (two possible classes and two possible groups) is four subsets of the dataset D. The subsets are labelled M1, M2, m1 and m2. The result of step S210 is illustrated in Figure 7, which is a graph showing the points of the dataset D after splitting into the four subsets. Figure 7 illustrates the datapoints of the training dataset across two attributes labeled $X_1$ and $X_2$ (which may for example be glucose level and BMI, or any other such attribute). Each datapoint corresponds to a data instance, i.e. to a human subject. The legend indicates to which subset each point corresponds. "+" represents the class of positive test result for diabetes which in this case is the majority class, "-" represents the class of negative test result for diabetes, "Group 1" corresponds to male and "Group 2" corresponds to female.

Step S220 comprises identifying the majority and minority classes for each group. In this case, as noted above the subsets corresponding to the majority class are M1 and M2 and the subsets corresponding to the minority class are m1 and m2.

Step S231 comprises splitting M1 into three random non-overlapping sets of points, Si, which may be referred to as auxiliary subsets. Step S232 comprises splitting M2 into three random non-overlapping sets of points, Ti, which may also be referred to as auxiliary subsets. In steps S231 and S232, the auxiliary subsets may all have the same size or they may be sized so as to be proportional to the other subsets. That is, the auxiliary subsets Si may be sized so as to be proportional to the subsets m1, m2 and M2, and the auxiliary subsets Ti may be sized so as to be proportional to the subsets m1, m2 and M1.

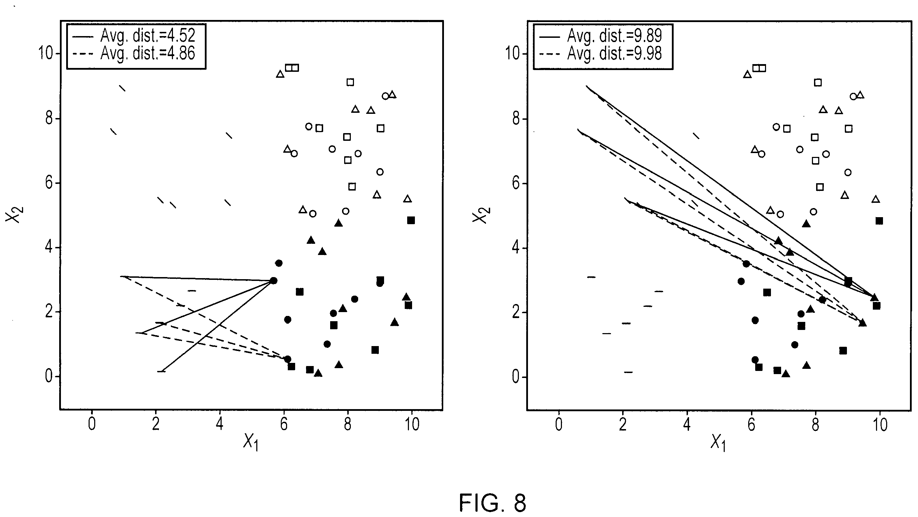

Figure 8 illustrates two graphs which show the points of the dataset D, similarly to Figure 7. In Figure 8, the auxiliary subsets Si and Ti are illustrated. That is, the subset M1 has been split into the auxiliary subsets Si indicated by the filled (solid) shapes, with each auxiliary subset Si indicated by a different shape (square, triangle, circle), and the subset M2 has been split into the auxiliary subsets Ti indicated by the unfilled shapes, with each auxiliary subset Ti indicated by a different shape (square, triangle, circle).

The splitting in steps S231 and S232 is done by a Random Splitter (uniform spitting). The reason for this is that each of the auxiliary subsets ought to follow the distribution of the original sets (M1 and M2) as much as possible.

The size of each of the auxiliary subsets may be considered as a hyperparameter of the process that can be tuned. As mentioned above two possible options are

o Uniform splitting (each of the three sets has the same size)
o Splitting proportional to the size of the other sets appearing in the problem. For example, the splitting of *M*1 will be proportional to the size of *M*2, *m*1, and *m*2.

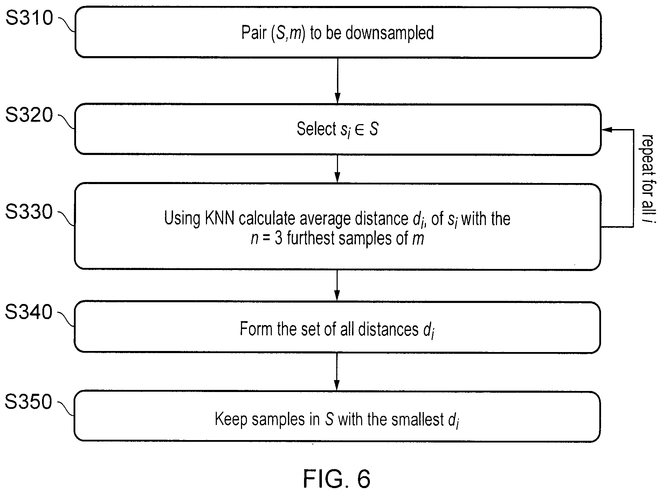

Steps S231 and S242 comprise downsampling the auxiliary subsets Si with respect to the subsets m1, m2 and M2, and downsampling the auxiliary subsets Ti with respect to the subsets m1, m2 and M1, respectively. These steps are described in more detail below with reference to Figure 6.

Figure 6 is a diagram illustrating a method of downsampling a given auxiliary subset with respect to a subset. Step S231 comprises performing the method of Figure 6 for each of the auxiliary subsets Si and Step S232 comprises performing the method of Figure 6 for each of the auxiliary subsets Ti.

The Figure 6 method is described for an auxiliary subset S. In step S310 the auxiliary subset S is selected for downsampling with respect to the corresponding set m. For example the auxiliary subset S may be S1 and the subset may be m1.

Step S320 comprises selecting a point si of the auxiliary subset S. Step S330 comprises calculating the average distance di to the n furthest datapoints of m. In this case, the k-nearest-neighbours (KNN) algorithm is used. The distance di is in a feature space or attribute space X1 and X2. That is, the distance di is in respect of the attributes X1 and X2. In this case, n=3, but this is not essential. In this step, the n furthest datapoints of the subset m are determined and then the average distance di is computed based on the distances of these n points from the point si.

The steps S320 and S330 are performed for all points of the auxiliary subset S.

Step S340 comprises forming the set of all distance di, i.e. the results of performing the steps S320 and S330 for all points of the auxiliary subset S. Step S350 comprises keeping the datapoints in S with the smallest average distance di. For example, a particular number of points are kept that have the smallest average distance di to their furthest n datapoints of the subset m. This step may also be considered as discarding a particular number of points having the largest average distance di to their furthest n datapoints of the subset m. The result of the step S350 is a downsampled auxiliary dataset S' corresponding to the auxiliary dataset S.

As mentioned above, the Figure 6 method is carried out for each auxiliary subset Si as part of step S241 and the Figure 6 method is carried out for each auxiliary subset Ti as part of step S242.

The results of steps S241 and S242 are downsampled auxiliary subsets Si' and Ti'. A downsampled training dataset D' may be generated/arranged by combining the downsampled auxiliary subsets and the subsets m1 and m2.

The graph in Figure 8 LHS illustrates the computation of average distances di for points of an auxiliary subset S with respect to the subset m1, and the graph in Figure 8 RHS illustrates the computation of average distances di for points of an auxiliary subset S with respect to the subset m2.

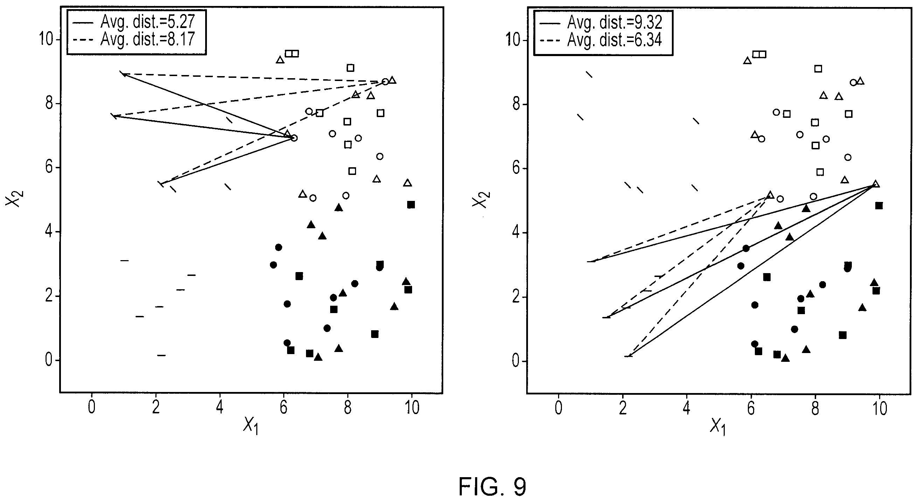

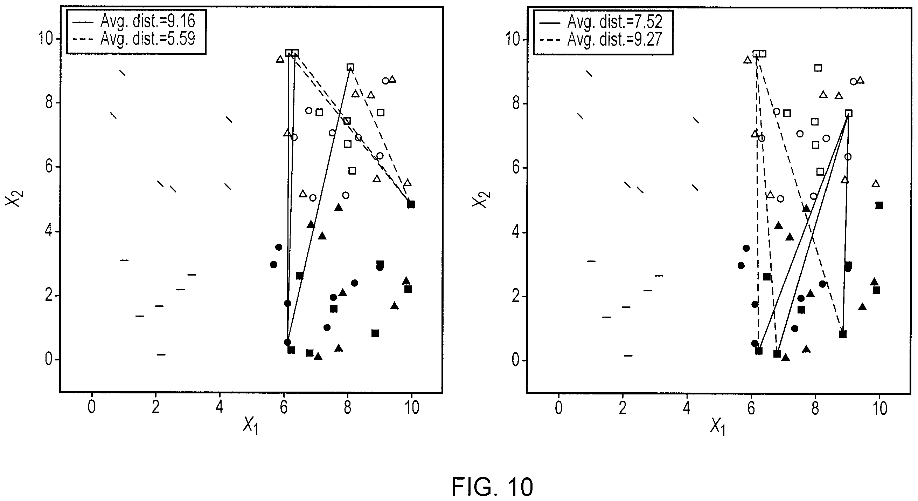

Figures 9 and 10 illustrate the datapoints of the dataset M split into the subsets and the auxiliary subsets in the same way as Figure 8.

The graph in Figure 9 LHS illustrates the computation of average distances di for points of an auxiliary subset T with respect to the subset m2, and the graph in Figure 9 RHS illustrates the computation of average distances di for points of an auxiliary subset T with respect to the subset m1.

The graph in Figure 10 LHS illustrates the computation of average distances di for points of an auxiliary subset T with respect to the subset M1, and the graph in Figure 10 RHS illustrates the computation of average distances di for points of an auxiliary subset S with respect to the subset M2.

By keeping the points which have the smallest average distance di to their k furthest points when downsampling a given auxiliary subset with respect to the subset it is paired with, the likelihood of keeping points close to a boundary between the auxiliary subset and the paired subset is increased, and points further away from the boundary are more likely to be discarded in the downsampling. The boundary may e.g. relate to a decision boundary with respect to an ML model which is trained using the downsampled training dataset. As described above, this is done for each auxiliary subset with respect to the other subsets, so that the subsets corresponding to the majority class are downsampled but points are retained which are important for forming decision boundaries between different subsets in the ML model's learning.

The number of points discarded is a parameter which may be chosen and tuned.

Although the method described above involved only the two attributes X1 and X2, any number of attributes may be considered. That is, each datapoint/record may be defined by a plurality of attributes, e.g. more than two. The above method was described considering an example in which the class related to the presence of absence of diabetes, and the groups were male and female, however this is not essential and the data may relate to other scenarios.

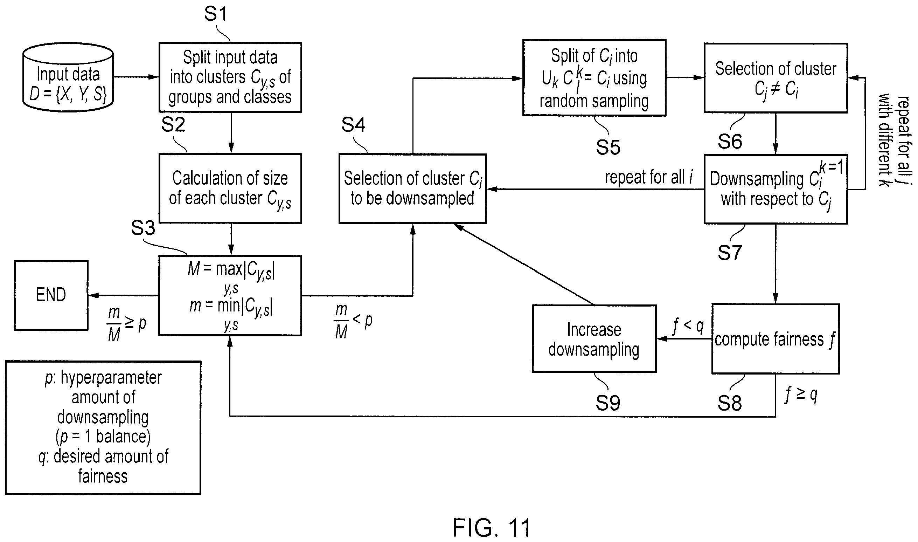

Figure 11 is diagram illustrating a method. The Figure 11 method may be considered to correspond to the method described above with respect to Figures 5 and 6.

At step S1, input data D = {X, Y, S} is split into clusters/subsets $C_{y,s}$ based on the group and class to which the records belong/correspond. The datapoints/records of the dataset D are defined by the attributes X, the classes Y and the groups S, similarly to the dataset described with respect to Figure 5. For example, if there are two possible classes and two possible groups, there will be four clusters $C_{y,s}$. If there are two possible classes and three possible groups, or three possible classes and two possible groups, there will be six clusters $C_{y,s}$. As previously described, a protected attribute gives rise to groups. There may be more than one protected attribute, for example two. In this case the dataset may be considered to be defined as D = {X, Y, S, S^}, where S and S^ correspond to the two protected attributes. If there are two classes, and if each protected attribute S, S^ gives rise to two groups, there will be eight subsets $C_{y,s}$.

Step S2 comprises calculating the size of each cluster $C_{y,s}$ in terms of the number of datapoints therein.

Step S3 comprises finding the largest and the smallest cluster and computing a ratio between the number of points in the largest cluster and the number of points in the smallest cluster. The ratio is compared with a hyperparameter p which indicates a desired amount of downampling. If the ratio meets a threshold related to the hyperparameter p then the method ends. If the ratio does not meet a threshold related to the hyperparameter p, then the method proceeds to step S4. The step S3 may be considered as checking whether downsampling is needed, that is, if the dataset D is imbalanced to an extend that downsampling is needed.

Step S4 comprises selecting a cluster to be downsampled. The cluster to be downsampled Ci is selected from among the clusters corresponding to a particular class, preferably the majority class (if a majority class exists).

Step S5 comprises splitting the cluster Ci into auxiliary subsets $C_i^k$, where $U_k C_i^k = Ci$. As in the Figure 5 method, the number of auxiliary subsets the cluster Ci is split into depends on the total number of clusters. That is, the cluster Ci is split into h auxiliary subsets, where h+1 is the number of clusters.

Step S6 comprises selecting a cluster Cj, where j is different to i. That is, a cluster other than the cluster currently selected for downsampling is selected. Step S7 comprises downsampling an auxiliary subset $C_i^k$ with respect to the cluster Cj. Downsampling the auxiliary subset with respect to the cluster comprises retaining a number of points of the auxiliary dataset with the smallest average distance to the k furthest points of the cluster, as described above with respect to Figure 6.

The method comprises repeating the steps S6 and S7 so that steps S6 and S7 are performed for all the clusters Cj apart from the cluster Ci which is currently selected for downsampling. Each time step S7 is performed a different auxiliary subset $C_i^k$ is selected (i.e. a different k). It may be that the auxiliary subsets are paired with the clusters other than the cluster from which the auxiliary subsets are divided, as in the Figure 5 method.

It may be that the auxiliary subsets are generated with sizes proportional to the clusters other than the cluster from which the auxiliary subsets are divided - alternatively, the auxiliary subsets may be generated all having the same size.

The steps S4-S7 are repeated for all the clusters to be downsampled, i.e. all the clusters corresponding to the majority class, and the method then proceeds to step S8. Step S8 comprises computing fairness, or a fairness measure, and comparing the fairness measure with a threshold fairness measure. If the fairness measure meets the fairness measure threshold, the method proceeds to step S3. If the fairness measure does not meet the fairness measure threshold, the method proceeds to step S9 and then step S4 and the downsampling is carried out again for each cluster to be downsampled.

That is, when repeating steps S4-S7 after step S9, downsampling according to steps S4-S7 is performed for the downsampled clusters resulting from the previous iteration of the steps S4-S7. Each downsampled cluster comprises the downsampled auxiliary subsets corresponding to the auxiliary susbets divided from the cluster corresponding to the downsampled cluster. Therefore the step S5 may not be carried out on repetitions of the steps S4-S7. Alternatively, the downsampling process may be carried out again based on the original auxiliary subsets but a different (e.g. smaller) number of points may be retained in each case - in this way, the step S5 need not be performed again. Alternatively the steps S5-S7 may all be carried out again based on the original clusters but a different (e.g. smaller) number of points may be retained in each case - in this way, step S5 is carried out again, and the auxiliary subsets are not necessarily the same as the auxiliary subsets generated when the steps S5-S7 were performed the first time. In the case in which a different (e.g. smaller) number of points are retained in each case of downsampling an auxiliary subset, the step S9 may comprise changing (e.g. increasing) the amount of downsampling - i.e. changing (decreasing) the number of points to be retained in the case of downsampling each auxiliary subset.

After the end of the method, a downsampled training dataset D' may be generated/arranged by combining the downsampled auxiliary subsets and the clusters which were not among clusters to be downsampled (i.e. the clusters corresponding to the minority class(es)).

Considering Figures 5/6 and 11, it will be appreciated that step S1 may be considered to correspond to step S210, steps S2 & S3 to step S220, step S5 to steps S231 & S232, and step S7 to steps S241 & S242. Description of any given step in the Figure 5/6 or Figure 11 method may be applied to corresponding step(s) in the other method.

The following fairness measures are described below: statistical parity difference, disparate impact, equality of opportunity difference, equality of opportunity ratio, average odds difference, and average odds ratio. The fairness measure computed in step S8 may comprise any of these.

Demographic parity is based on the principle that the groups should receive an equal proportion of positive labels. This can be monitored by the fairness measures statistical parity difference and/or disparate impact.

Statistical parity difference: f = Prob (Y'=1 | A=0) - Prob (Y'=1 | A=1)

Disparate impact: f = Prob (Y'=1 | A=0) = Prob (Y'=1 | A=1)

where the true positive rate Prob (Y'=1) is defined as

$$\text{Prob (Y'=1)} = [TP + FP] \div [P + N],$$

where TP is true positive, FP is false positive, P is total positives (TP+FP), and N is total negatives (true negatives TN + false negatives FN), and where Y means the actual class of the data record concerned and Y' means the predicted class of the data record concerned. A=0 and A=1 refers to two different groups.

Equality of opportunity is based on the principle that the groups should have an equal true positive rate (TPR).

Equality of opportunity difference: f = Prob (Y'=1 | A=0, Y=1) - Prob (Y'=1 | A=1, Y=1)

Equality of opportunity ratio: f = Prob (Y'=1 | A=0, Y=1) ÷ Prob (Y'=1 | A=1, Y=1)

Average odds is based on the principle that the groups should have equal TPR and equal false positive rate (FPR).

Average odds difference:

$$f = \tfrac{1}{2} * [ \ | \ \text{Prob} \ (Y'=1 \mid A=0, \ Y=1) - \text{Prob} \ (Y'=1 \mid A=1, \ Y=1) \ |$$

$$+$$

$$| \ \text{Prob} \ (Y'=1 \mid A=0, \ Y=0) - \text{Prob} \ (Y'=1 \mid A=1, \ Y=0) \ | \ ]$$

Average odds ratio:

$$f = \tfrac{1}{2} * [ \ \text{Prob} \ (Y'=1 \mid A=0, \ Y=1) \div \text{Prob} \ (Y'=1 \mid A=1, \ Y=1)$$

$$+$$

$$\text{Prob} \ (Y'=1 \mid A=0, \ Y=0) \div \text{Prob} \ (Y'=1 \mid A=1, \ Y=0) \ ]$$

It will be appreciated that the above fairness measures make use of predictions of an ML model. Therefore the step S8 may include training an ML model using the downsampled dataset, where the ML model is configured to predict the class of a given data record based on the attributes/features X (but not based on the attribute which dictates the group(s) to which the record belongs), and evaluating the trained ML model using testing data to acquire the prediction of class Y' for each record of the testing data, so that the above quantities (TPR, FPR, etc., may be computed).

The above examples of fairness measures assume one protected attribute giving rise to two groups, and two possible classes. In the case of a protected attribute giving rise to more than two groups, a fairness measure as described above may be computed in respect of any two groups, or a fairness measure may be computed as described above in respect of each combination of two groups and the fairness measures may be combined to generate an overall fairness measure. In case there is more than protected attribute, a fairness measure may be computed in respect of each protected attribute and these fairness measures may be combined to generate an overall fairness measure.

In the case of more than two classes, any of the above fairness measures may be utilized e.g. for one class compared to each other class and then combined. It will be appreciated that there are many metrics for evaluating fairness in the case of binary classification problems and in the case of more than two classes (for example, https://www. evidentlyai.com/classification-metrics/multi-class-metrics) and the above metrics are described above as examples with respect to a binary classification problem. For instance, a true positive rate (TPR) and false positive rate (FPR) may be computed for each class separately (TPR_class_i = TP class_i / [TP_class_i + FN_class_i]), and from such quantities many fairness metrics may be computed (e.g. average TPR or FPR across all classes).

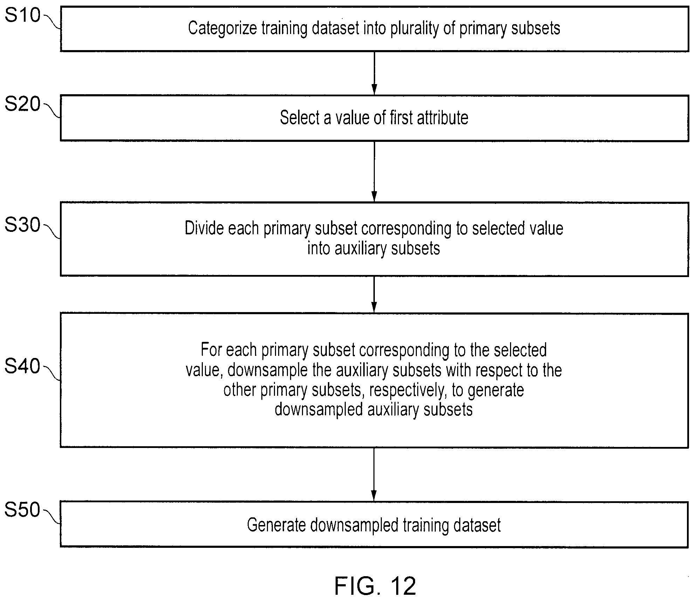

Figure 12 is a diagram illustrating a method. The Figure 12 method may be considered to correspond to the methods of Figures 5/6 and 11. The Figure 12 method comprises steps S10, S20, S30, S40, and S50.

Step S10 comprises categorizing a training dataset into a plurality of primary subsets. That is, step S10 comprises categorizing each datapoint in the training dataset into one of a plurality of primary subsets based on values of the datapoint in respect of at least first and second attributes. Each primary subset corresponds to specific values in respect of at least the first and second attributes. After the categorization, for each primary subset the datapoints in the subset share the same value for the first attribute and the same value for the second attribute. Each datapoint is defined by values in respect of a plurality of attributes including the at least first and second attributes.

Step S20 comprises selecting a value of the first attribute. That is, step S20 comprises selecting a specific value in respect of the first attribute. This may be the value of the first attribute which is most common among the training dataset (the majority value). If there is no imbalance in the dataset with respect to the first attribute, then a value of the first attribute may be randomly selected.

Step S30 comprises dividing each primary subset corresponding to the selected value into auxiliary subsets. That is, step S30 comprises dividing each of the primary subsets corresponding to the selected value in respect of the first attribute into a plurality of auxiliary subsets.

Steo S40 comprises, for each primary subset corresponding to the selected value, downsampling the auxiliary subsets with respect to the other primary subsets, respectively, to generate downsampled auxiliary subsets. That is, step S40 comprises for each of the primary subsets corresponding to the selected value in respect of the first attribute, downsampling the plurality of auxiliary subsets with respect to the other primary subsets, respectively, to generate a plurality of downsampled auxiliary subsets. For a given primary subset corresponding to the selected value, each auxiliary subset may be considered to correspond to one of the other primary subsets.

Step S50 comprises generating a downsampled training dataset. That is, Step S50 comprises generating a downsampled training dataset for training an ML model to predict (a value of) the first attribute, the downsampled training dataset comprising the datapoints of the downsampled auxiliary subsets and the datapoints of the primary subsets other than the primary subsets corresponding to the selected value in respect of the first attribute.

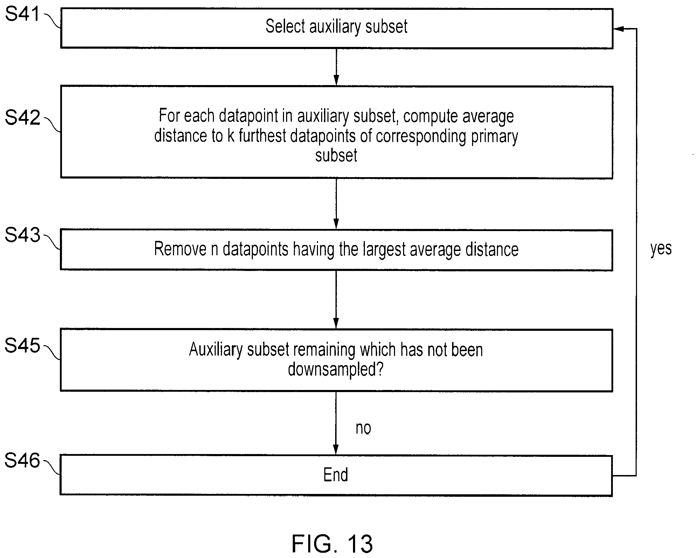

The downsampling in step S40 comprises the steps S41-S46 illustrated in the Figure 13 method. Step S41 comprises selecting an auxiliary subset. Step S42 comprises, for each datapoint in the auxiliary subset, computing the average distance to the k furthest datapoints of the corresponding primary subset (i.e. the primary subset with respect to which the auxiliary subset concerned is being downsampled). That is, step S42 comprises, for each datapoint in the auxiliary subset concerned, computing an average distance to the k furthest datapoints of the primary subset concerned in respect of the plurality of attributes other than the at least first and second attributes (i.e. in a feature space of the plurality of attributes other than the at least first and second attributes).

Step S43 comprises removing the n datapoints having the largest average distance. That is, step S43 comprises removing from the auxiliary subset concerned n datapoints of the auxiliary subset concerned having the largest computed average distance (to their respective k furthest datapoints of the primary subset concerned) to generate the downsampled auxiliary subset concerned, k and n are positive integers.

Step S43 may be considered to comprise selecting n' datapoints of the auxiliary subset concerned having the smallest computed average distance (to their respective k furthest datapoints of the primary subset concerned) for inclusion in the downsampled auxiliary subset concerned. Depending on the choice of n and n', this amounts to the same process as the description of step S43 in the above paragraph.

Step S45 comprises determining whether any auxiliary subset remains which has not been downsampled. If there is, then the method returns to step S41 and that auxiliary subset is selected. If there is not, the method ends.

There may be more than two possible values of the first attribute - that is, considering the values the first attribute as "classes", there may be more than two classes. In this case, a single class may be selected and the method may be the same as described above, or another class may be selected in addition and then division into auxiliary subsets and downsampling carried out in the same way as defined in the steps S30 and S40 for the other class. Any number of classes may be selected (for example the w most common classes, where w is a positive integer) and division into auxiliary subsets and downsampling carried out in the same way as defined in the steps S30 and S40 for the selected classes. The same considerations apply to the Figures 5/6 and 11 methods, in the sense that where there are more than two classes, any number of classes may be selected (for example the w most common classes, where w is a positive integer) and division into sets Si/Ti (which may be referred to as auxiliary subsets) and downsampling carried out in the same way as defined in the corresponding steps of those methods.

As described above, there is disclosed herein a computer-implemented method comprising: categorizing each datapoint in a training dataset into one of a plurality of primary subsets based on values of the datapoint in respect of (at least) first and second attributes (so that each primary subset corresponds to specific values (in respect) of (at least) the first and second attributes) (so that for each primary subset the datapoints in the subset share the same value for the first attribute and the same value for the second attribute), (wherein each datapoint is defined by (values in respect of) a plurality of attributes including the at least first and second attributes); selecting a specific value in respect of the first attribute and dividing each of the primary subsets corresponding to the selected value (in respect) of the first attribute into a plurality of auxiliary subsets; for each of the primary subsets corresponding to the selected value (in respect) of the first attribute, downsampling the plurality of auxiliary subsets with respect to the other primary subsets, respectively, to generate a plurality of downsampled auxiliary subsets; and generating a downsampled training dataset for training a machine learning, ML, model to predict (a value of) the first attribute, the downsampled training dataset comprising the datapoints of the downsampled auxiliary subsets and the datapoints of the primary subsets other than the primary subsets corresponding to the selected value (in respect) of the first attribute, wherein the downsampling comprises: for each datapoint in the auxiliary subset concerned, computing an average distance to the k furthest datapoints of the primary subset concerned (the distance) in respect of the plurality of attributes other than the at least first and second attributes; and removing (from the auxiliary subset concerned) n datapoints of the auxiliary subset concerned having the largest computed average distance (to their respective k furthest datapoints of the primary subset concerned) to generate the downsampled auxiliary subset concerned, where k and n are positive integers.

The second attribute may be a protected attribute.

The ML model may be configured to predict the first attribute based on the plurality of attributes other than at least the second attribute.

The first attribute for a given datapoint may comprise one of two values.

The ML model may be configured to classify a datapoint in respect of the first attribute.

The ML model may be configured to classify a datapoint in respect of the first attribute, the classification comprising a binary classification.

Selecting the specific value in respect of the first attribute may comprise, when the training dataset is imbalanced in respect of the first attribute, selecting a majority value in respect of the first attribute, the majority value being the most common value among the training dataset in respect of the first attribute.

Selecting the specific value in respect of the first attribute may comprise, when there exists a majority value in respect of the first attribute, selecting the majority value in respect of the first attribute, the majority value being the most common value among the training dataset in respect of the first attribute.

Selecting the specific value in respect of the first attribute may comprise, when the training dataset is imbalanced in respect of the first attribute, selecting the most common value among the training dataset in respect of the first attribute.

The computer-implemented method may comprise selecting at least one other value of the first attribute and for each selected at least one other value of the first attribute, dividing each of the primary subsets corresponding to the selected other value of the first attribute into a plurality of further auxiliary subsets and downsampling the further auxiliary subsets, wherein the downsampled training dataset comprises the datapoints of the downsampled auxiliary subsets and the datapoints of the downsampled further auxiliary subsets and (the) datapoints of the primary subsets other than the primary subsets corresponding to the selected value of the first attribute and the primary subsets corresponding to the selected at least one other value of the first attribute.

Selecting the at least one other value of the first attribute may comprise selecting the at least one next most common value of the first attribute after the selected specific value among the training dataset.

Dividing each of the primary subsets corresponding to the selected value in respect of the first attribute into a plurality of auxiliary subsets may comprise, for each primary subset corresponding to the selected value in respect of the first attribute, dividing the primary subset into h auxiliary subsets, wherein h+1 is the number of primary subsets.

Dividing each of the primary subsets corresponding to the selected value in respect of the first attribute into a plurality of auxiliary subsets may comprise, for each primary subset corresponding to the selected value in respect of the first attribute, dividing the primary subset into a number of auxiliary subsets equal to the number of other primary subsets.

For each of the primary subsets corresponding to the selected value in respect of the first attribute, each auxiliary subset may correspond to one of the other primary subsets, and the downsampling of each auxiliary subset may be carried out with respect to the corresponding other primary subset.

For each of the primary subsets corresponding to the selected value in respect of the first attribute, the auxiliary subsets may correspond respectively to the other primary subsets, and the downsampling of each auxiliary subset may be carried out with respect to the corresponding other primary subset.

Dividing each of the primary subsets corresponding to the selected value in respect of the first attribute into a plurality of auxiliary subsets may comprise dividing each primary subset uniformly.

Dividing each of the primary subsets corresponding to the selected value in respect of the first attribute into a plurality of auxiliary subsets may comprise, for each primary subset corresponding to the selected value in respect of the first attribute, dividing the primary subset into the plurality of auxiliary subsets sized corresponding/proportionally to the other primary subsets, respectively.

For each of the primary subsets corresponding to the selected value in respect of the first attribute, the corresponding auxiliary subsets may be sized corresponding/proportionally to the other primary subsets, respectively.

For each of the primary subsets corresponding to the selected value in respect of the first attribute, the corresponding auxiliary subsets may comprise a number of datapoints corresponding/proportional to the other primary subsets, respectively.

Dividing each of the primary subsets corresponding to the selected value in respect of the first attribute into a plurality of auxiliary subsets may comprise using random sampling.

Generating the donwsampled training dataset may comprise combining the downsampled auxiliary subsets and the primary subsets other than the primary subsets corresponding to the selected value (in respect) of the first attribute.

For each datapoint in the auxiliary subset concerned, computing the average distance to the k furthest datapoints of the primary subset concerned may comprise computing the distance to each datapoint of the primary subset concerned and determining the k furthest datapoints.

The average distance may comprise a Euclidean distance.

For each datapoint in the auxiliary subset concerned, computing the average distance to the k furthest datapoints of the primary subset concerned (, the distance) in respect of the plurality of attributes other than the at least first and second attributes may comprise computing the average distance in the feature/attribute space of the plurality of attributes other than the at least first and second attributes.

The at least first and second attributes may further comprise at least a third attribute.

Each of the second and third attribute may be a protected attribute.

The ML model may be configured to predict the first attribute based on the plurality of attributes other than at least the second and third attributes.

The computer-implemented method may comprise: computing a fairness measure of the downsampled training dataset; if the fairness measure fails to meet a threshold fairness, for each of the primary subsets corresponding to the selected value (in respect) of the first attribute further downsampling the plurality of auxiliary subsets with respect to the other primary subsets, respectively, to generate a plurality of further downsampled auxiliary subsets; and generating a further downsampled training dataset for training a machine learning, ML, model to predict (a value of) the first attribute, the downsampled training dataset comprising the datapoints of the further downsampled auxiliary subsets and the datapoints of the primary subsets other than the primary subsets corresponding to the selected value (in respect) of the first attribute.

The computer-implemented method may comprise iteratively downsampling the plurality of auxiliary subsets until the computed fairness measure meets the threshold fairness.

The fairness measure may comprise at least one of statistical parity difference, statistical parity ratio, equality of opportunity difference, equality of opportunity ratio, average/equalized odds difference, and average/equalized odds ratio.

The computer-implemented method may comprise: (after downsampling) computing a ratio between the largest of the primary subsets and the smallest of the primary subsets; when the ratio fails to meet a threshold ratio, for each of the primary subsets corresponding to the selected value (in respect) of the first attribute further downsampling the plurality of auxiliary subsets with respect to the other primary subsets, respectively, to generate a plurality of further downsampled auxiliary subsets; and generating a further downsampled training dataset for training the ML model to predict (a value of) the first attribute, the further downsampled training dataset comprising the datapoints of the further downsampled auxiliary subsets and the datapoints of the primary subsets other than the primary subsets corresponding to the selected value (in respect) of the first attribute.

The computer-implemented method may comprise iteratively downsampling the plurality of auxiliary subsets until the ratio meets the threshold ratio.

The training dataset may comprise medical data.

The training dataset may comprise medical data and wherein each datapoint of the training dataset relates to a human subject or patient.

The second attribute is any one of gender, race, religion, ethnicity, age (group), sex, presence of pregnancy, presence of a disability, presence of gender reassignment, marriage, civil partnership, sexual orientation.

The first attribute may be the presence of a disease or condition.

The first attribute may be the likelihood of current or future presence of a disease or condition.

The second attribute may be gender and the first attribute may be the presence of diabetes.

The second attribute may be gender and the first attribute may be the presence of a disease or condition.

The ML model may be configured to predict (a value of) the first attribute on the basis of the plurality of attributes other than the at least first and second attributes.

The computer-implemented method may further comprise training the ML model using the downsampled training dataset.

The computer-implemented method may further comprise using the ML model to predict (a value of) the first attribute in respect of a new data instance.

The computer-implemented method may further comprise using the ML model to predict (a value of) the first attribute in respect of a new human subject or patient.

The computer-implemented method may further comprise using the ML model to predict the presence or absence of a disease.

The computer-implemented method may further comprise outputting a diagnosis in respect of the new human subject or patient comprising the prediction of the presence or absence of the disease.

The computer-implemented method may further comprise using the ML model to predict the likelihood of current or future presence of a disease or condition.

The training dataset may comprise medical data and each datapoint of the training dataset may relate to a human subject or patient, the first attribute may be the presence of a disease or condition, the computer-implemented method may comprise training the ML model using the downsampled training dataset and using the ML model to predict [a value of] the first attribute in respect of a new human subject or patient, and the computer-implemented method may comprise outputting a diagnosis in respect of the new human subject or patient comprising the prediction of the presence or absence of the disease or condition.

There is disclosed herein a computer program (comprising instructions) which, when run on a computer, causes the computer to carry out a method comprising: categorizing each datapoint in a training dataset into one of a plurality of primary subsets based on values of the datapoint in respect of (at least) first and second attributes (so that each primary subset corresponds to specific values (in respect) of (at least) the first and second attributes) (so that for each primary subset the datapoints in the subset share the same value for the first attribute and the same value for the second attribute), (wherein each datapoint is defined by (values in respect of) a plurality of attributes including the at least first and second attributes); selecting a specific value in respect of the first attribute and dividing each of the primary subsets corresponding to the selected value (in respect) of the first attribute into a plurality of auxiliary subsets; for each of the primary subsets corresponding to the selected value (in respect) of the first attribute, downsampling the plurality of auxiliary subsets with respect to the other primary subsets, respectively, to generate a plurality of downsampled auxiliary subsets; and generating a downsampled training dataset for training a machine learning, ML, model to predict (a value of) the first attribute, the downsampled training dataset comprising the datapoints of the downsampled auxiliary subsets and the datapoints of the primary subsets other than the primary subsets corresponding to the selected value (in respect) of the first attribute, wherein the downsampling comprises: for each datapoint in the auxiliary subset concerned, computing an average distance to the k furthest datapoints of the primary subset concerned (the distance) in respect of the plurality of attributes other than the at least first and second attributes; and removing (from the auxiliary subset concerned) n datapoints of the auxiliary subset concerned having the largest computed average distance (to their respective k furthest datapoints of the primary subset concerned) to generate the downsampled auxiliary subset concerned, where k and n are positive integers.

There is disclosed herein an information processing apparatus comprising a memory and a processor connected to the memory, wherein the processor is configured to: categorize each datapoint in a training dataset into one of a plurality of primary subsets based on values of the datapoint in respect of (at least) first and second attributes (so that each primary subset corresponds to specific values (in respect) of (at least) the first and second attributes) (so that for each primary subset the datapoints in the subset share the same value for the first attribute and the same value for the second attribute), (wherein each datapoint is defined by (values in respect of) a plurality of attributes including the at least first and second attributes); select a specific value in respect of the first attribute and dividing each of the primary subsets corresponding to the selected value (in respect) of the first attribute into a plurality of auxiliary subsets; for each of the primary subsets corresponding to the selected value (in respect) of the first attribute, downsample the plurality of auxiliary subsets with respect to the other primary subsets, respectively, to generate a plurality of downsampled auxiliary subsets; and generate a downsampled training dataset for training a machine learning, ML, model to predict (a value of) the first attribute, the downsampled training dataset comprising the datapoints of the downsampled auxiliary subsets and the datapoints of the primary subsets other than the primary subsets corresponding to the selected value (in respect) of the first attribute, wherein the downsampling comprises: for each datapoint in the auxiliary subset concerned, computing an average distance to the k furthest datapoints of the primary subset concerned (the distance) in respect of the plurality of attributes other than the at least first and second attributes; and removing (from the auxiliary subset concerned) n datapoints of the auxiliary subset concerned having the largest computed average distance (to their respective k furthest datapoints of the primary subset concerned) to generate the downsampled auxiliary subset concerned, where k and n are positive integers.

It will be appreciated that the step S10 may be considered to correspond to step S210 and/or step S1, step S20 to step S220 and/or step S4, step S30 to Steps S231 & S232 and/or step S5, step S40 to Steps S241 & S242 and/or steps S6 & S7, step S41 to Step S310, step S42 to step S330, and step S43 to step S350. Description of any given step in the Figure 5/6 or Figure 11 or Figure 12/13 method may be applied to corresponding step(s) in any other method.

Any of the Figure 5/6 or Figure 11 or Figure 12/13 methods may be applied to a training dataset comprising medical data and any of these methods may further comprise training the ML model using the downsampled training dataset and optionally using the trained ML model to predict the presence or absence of a disease, as described above with respect to the Figure 12/13 method. A diagnosis may be output based on the prediction.

Figure 14 illustrates results of evaluating ML models which have been trained separately on a dataset with no downsampling and datasets to which different downsampling procedures have been applied.

The dataset used as the original dataset is the Adult dataset (Adult - UCI Machine Learning Repository (https://archive.ics.uci.edu/dataset/2/adult)), a standard benchmark dataset. The ML models classified the data as true or false, which in the context of the Adult dataset means whether the income associated with a record is above a threshold amount or not. Each graph in Figure 14 shows results of applying eight ML models (classification algorithms) on three datasets. The circles in the top and bottom graphs indicate results of applying the ML models on the original dataset, and the triangles in the top and bottom graphs indicate the results of applying the ML models on the dataset after fair downsampling (i.e. downsampling in line with the Figures 5/6, 11, and 12/13 methods). The squares in the top three graphs indicate the results of applying the ML models on the dataset after downsampling according to the method described below referred to as a modified comparative method, and the squares in the bottom three graphs indicate the results of applying the ML models on the dataset after "random fair downsampling" - i.e. downsampling by splitting the dataset into clusters/subsets based on group and class but then using random downsampling on each "majority class" subset instead of using the distance-based approach of the "fair downsampling". For all downsampling approaches in obtaining the Figure 14 results the "amount of downsampling" was 0.5, meaning that half of the points corresponding to the majority class were discarded.

The ML models (classifiers) evaluated are

- LogisticRegression (https://scikit-learn.org/stable/modules/generated/sklearn.linear_model.LogisticRegression.html),
- SVC (https://scikit-learn.org/stable/modules/generated/sklearn.svm.SVC.html),
- RandomForestClassifier (https://scikit-learn.org/stable/modules/generated/sklearn.ensemble. RandomForestClassifier. html),
- KNeighborsClassifier (https://scikit-learn. org/stable/modules/generated/sklearn.neighbors.KNeighborsClassifier.ht ml),
- XGBClassifier (https://github.com/dmlc/xgboost),
- GaussianNB (https://scikit-learn.org/stable/modules/generated/sklearn.naive_bayes.GaussianNB.html),
- GradientBoostingClassifier (https://scikit-learn.org/stable/modules/generated/sklearn.ensemble.GradientBoosting Classifi er.html),
- DecisionTreeClassifier (https://scikit-learn.org/stable/modules/generated/sklearn.tree.DecisionTreeClassifier. html).

The LHS graphs show statistical parity difference against accuracy, the middle graphs show equal opportunity difference against accuracy, and the RHS graphs show average odds difference against accuracy. Accuracy was measured using a metric called 'balanced accuracy', which is appropriate dealing with imbalanced datasets:

$$\text{balanced accuracy} = \tfrac{1}{2} \left( TP / (TP + FN) + TN / (TN + FP) \right).$$

It will be appreciated that the fair downsampling improved the fairness of the classifier in most if not all cases, where the fairness is measured by any of the quantities shown on the y axes in the graphs in Figure 14. Furthermore, accuracy in general tends to increase . The measure of statistical parity shows the biggest improvement when fair downsampling is used compared to no downsampling or random downsampling.

Some general observations may be made (results of course depend somewhat on the datasets used and the following observations are an indication and a tendency, not a rule):

When a dataset is downsampled using Random Downsampling (comparative method):

a) There is a trade-off between accuracy and fairness.
b) After downsampling the accuracy increases most of the time by -1-5%. But sometimes it might decrease by a small amount.
c) After downsampling fairness reduces almost always by -5-20%.

When a dataset is downsampled using Fair downsampling (i.e. downsampling in line with the Figures 5/6, 11, and 12/13 methods):

a) A considerable improvement in fairness may be seen compared to traditional downsampling methods. This is more apparent in the case of "statistical parity difference" as shown in Figure 14 LHS graphs, where fairness might increase by 40% in some cases.

b) Accuracy is at least not diminished substantively, and in some cases it increases compared to the accuracy before downsampling. The accuracy gains are comparable with "random downsampling".

Aspects disclosed herein include the following method:

1. Split a given dataset D into groups $G_i$ and classes Ci. Consider a binary classification problem with one protected group (generalization to more than one protected groups is straightforward).
2. Let M1 and M2 be the majority classes for groups 1 and 2 respectively
3. Let m1 and m2 be the minority classes for groups 1 and 2 respectively
4. Split M1 into 3 random non-overlapping sets, S1 , S2 , S3. The union of the three sets is $M1$. Take the size of each of the three sets to be proportional to the size of $m1$ , $m2$, $M2$ respectively.
5. Downsample $M1$ in the pairs $(S1 , m1)$, $(S2 , m2)$, $(S3 , M2)$ . For the pair $(S1 , m1)$, downsampling of S1 is as follows. Calculate the average distance of each of the samples in $S1$ with the $n = 3$ furthest samples of $m1$. For this step the KNN algorithm is used. Keep only the samples in $S1$ with the smallest average distance. S2 and S3 are downsampled in the same way with respect to m2 and M2, respectively.
6. Similarly, split $M2$ into 3 random sets and downsample with respect to $m1$ , $m2$, $M1$ .

The comparative method described above with respect to Figure 2, which may be referred to hereinafter as Random Downsampling, involves randomly deleting samples from the majority class. It does not consider the boundaries between classes or groups. Therefore, it may lead to deletion of samples near the boundaries with the same probability as samples from the interior. This will affect the decision boundaries and consequently the accuracy and fairness of the resulting classifier trained on the downsampled dataset. This may lead to underfitting.

A naive approach to overcome these problems may be to keep more samples near the boundaries by employing a distance based downsampling approach, which is referred to herein and above as a modified comparative method. In this case samples are retained that have the smallest average distance from the $n = 3$ furthest points of the minority class. However this naive approach does not account for decision boundaries with respect to groups, e.g. privileged and unprivileged groups. The computational time/load is large and increases with the size of the datasets (because with more datapoints many more distances between points must be computed). The memory required for such an operation is large.

In the fair downsampling methodology disclosed herein (i.e. the Figures 5/6, 11, 12.13 methods), by considering all possible boundaries between subsets categorized based on group and class during downsampling, the size of the dataset is reduced without sacrificing fairness and accuracy. In fact, fairness is improved compared to traditional downsampling approaches, whereas accuracy does not substantively decrease and may increase, as described above. Consideration of boundaries related to the different group and class combinations overcomes the problem of underfitting.

Furthermore, the amount of memory/processing power/computing load/computing time required is reduced when using the fair downsampling methodology disclosed herein (i.e. the Figures 5/6, 11, 12.13 methods) compared to the naive approach involving KNN (especially when the dataset is large (~100k samples/records)), because subsets (e.g. $M1$ and $M2$) are split into the auxiliary subsets which have smaller sizes and these auxiliary subsets are each compared with one other subset and therefore fewer distances between pairs of points need to be considered, compared with e.g. the modified comparative method. Thus using the methodology disclosed herein it is possible to downsample larger datasets with the same memory/processing power/computing load/computing time. Compared to the naive approach involving the use of KNN (modified comparative method), a faster performance is achieved using the methodology disclosed herein.

Medical applications (e.g. outputting a prediction of the presence or absence of a disease) have been described above as applications of the ML model for which the training data is downsampled. Other scenarios are of course applicable, for example credit lending analysis (described below), performance evaluation of employees (described below), shortlisting candidates for school admission (protected attributes: parent's education level, postcode) or shortlisting candidates for jobs (protected: gender), etc.

Credit lending analysis: the area of application concerns the banking sector. The bank would like to analyze the creditworthiness of a prospective borrower, for example how likely it is to repay the interest. Protected attributes can be for example nationality, marital status, education, gender.

Performance evaluation of employees: this is an imbalanced problem because only a few employees score excellent. Protected attributes: most often gender.

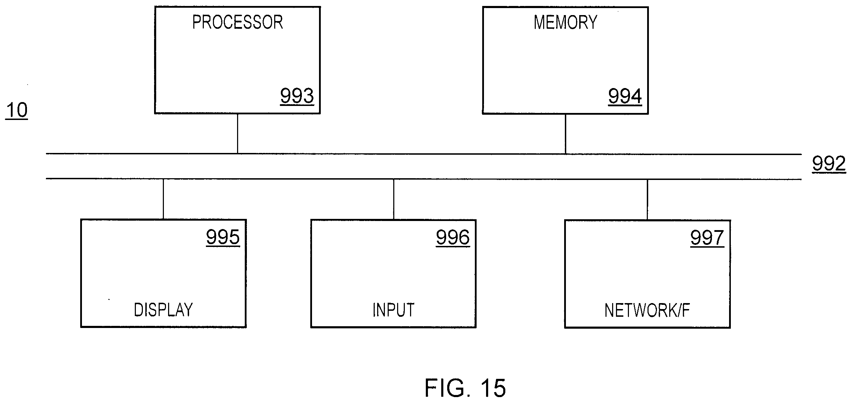

Figure 15 is a block diagram of an information processing apparatus 10 or a computing device 10, such as a data storage server, which embodies the present invention, and which may be used to implement some or all of the operations of a method embodying the present invention, and perform some or all of the tasks of apparatus of an embodiment. The computing device 10 may be used to implement any of the method steps described above, e.g. any of steps S110-S150, S210-S242, S310-S350, S1-S9, S10-S50, and S41-S46.

The computing device 10 comprises a processor 993 and memory 994. Optionally, the computing device also includes a network interface 997 for communication with other such computing devices, for example with other computing devices of invention embodiments. Optionally, the computing device also includes one or more input mechanisms such as keyboard and mouse 996, and a display unit such as one or more monitors 995. These elements may facilitate user interaction. The components are connectable to one another via a bus 992.

The memory 994 may include a computer readable medium, which term may refer to a single medium or multiple media (e.g., a centralized or distributed database and/or associated caches and servers) configured to carry computer-executable instructions. Computer-executable instructions may include, for example, instructions and data accessible by and causing a computer (e.g., one or more processors) to perform one or more functions or operations. For example, the computer-executable instructions may include those instructions for implementing a method disclosed herein, or any method steps disclosed herein, for example any of steps S110-S150, S210-S242, S310-S350, S1-S9, S10-S50, and S41-S46. Thus, the term "computer-readable storage medium" may also include any medium that is capable of storing, encoding or carrying a set of instructions for execution by the machine and that cause the machine to perform any one or more of the method steps of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 993 is configured to control the computing device and execute processing operations, for example executing computer program code stored in the memory 994 to implement any of the method steps described herein. The memory 994 stores data being read and written by the processor 993 and may store training data and/or ML model weights and/or information regarding protected attributes and/or subsets and/or auxiliary subsets and/or threshold information and/or formula(e) for computing fairness measures and/or distance measures and/or downsampled auxiliary subsets and/or downsampled training datasets and/or diagnosis information and/or ML model predictions and/or testing data and/or input data and/or other data, described above, and/or programs for executing any of the method steps described above. As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the operations and operations discussed herein. The processor 993 may be considered to comprise any of the modules described above. Any operations described as being implemented by a module may be implemented as a method by a computer and e.g. by the processor 993.

The display unit 995 may display a representation of data stored by the computing device, such as a representation of points to be retained/discarded in downsampling and/or ML model predictions and/or GUI windows and/or interactive representations enabling a user to interact with the apparatus 10 by e.g. drag and drop or selection interaction, and/or any other output described above, and may also display a cursor and dialog boxes and screens enabling interaction between a user and the programs and data stored on the computing device. The input mechanisms 996 may enable a user to input data and instructions to the computing device, such as enabling a user to input any user input described above.

The network interface (network I/F) 997 may be connected to a network, such as the Internet, and is connectable to other such computing devices via the network. The network I/F 997 may control data input/output from/to other apparatus via the network. Other peripheral devices such as microphone, speakers, printer, power supply unit, fan, case, scanner, trackerball etc may be included in the computing device.

Methods embodying the present invention (for example any of steps S110-S150, S210-S242, S310-S350, S1-S9, S10-S50, and S41-S46) may be carried out on a computing device/apparatus 10 such as that illustrated in Figure 15. Such a computing device need not have every component illustrated in Figure 15, and may be composed of a subset of those components. For example, the apparatus 10 may comprise the processor 993 and the memory 994 connected to the processor 993. Or the apparatus 10 may comprise the processor 993, the memory 994 connected to the processor 993, and the display 995. A method embodying the present invention may be carried out by a single computing device in communication with one or more data storage servers via a network. The computing device may be a data storage itself storing at least a portion of the data.

A method embodying the present invention may be carried out by a plurality of computing devices operating in cooperation with one another. One or more of the plurality of computing devices may be a data storage server storing at least a portion of the data.

The computing device 10 may have multiple processors or a processor logically separated into multiple virtual processors. Methods disclosed herein may be carried out using such multiple processors or virtual processors, or using a plurality of computing devices 10. The downsampling of auxiliary datasets may be carried out using multiple computing devices 10 or multiple processors or multiple virtual processors, in parallel. The multiple processors or virtual processors (or processors present in different computing devices 10) may have different sizes/capacities and the downsampling of auxiliary subsets may be assigned to the processors/virtual processors/computing devices according to the size of the auxiliary subsets, e.g. so that larger auxiliary datasets are downsampled (in parallel) using larger/higher capacity processors/virtual processors.

The invention may be implemented in digital electronic circuitry, or in computer hardware, firmware, software, or in combinations of them. The invention may be implemented as a computer program or computer program product, i.e., a computer program tangibly embodied in a non-transitory information carrier, e.g., in a machine-readable storage device, or in a propagated signal, for execution by, or to control the operation of, one or more hardware modules.

A computer program may be in the form of a stand-alone program, a computer program portion or more than one computer program and may be written in any form of programming language, including compiled or interpreted languages, and it may be deployed in any form, including as a stand-alone program or as a module, component, subroutine, or other unit suitable for use in a data processing environment. A computer program may be deployed to be executed on one module or on multiple modules at one site or distributed across multiple sites and interconnected by a communication network.

Method steps of the invention (for example any of steps S110-S150, S210-S242, S310-S350, S1-S9, S10-S50, and S41-S46) may be performed by one or more programmable processors executing a computer program to perform functions of the invention by operating on input data and generating output. Apparatus of the invention may be implemented as programmed hardware or as special purpose logic circuitry, including e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions coupled to one or more memory devices for storing instructions and data.

The above-described embodiments of the present invention may advantageously be used independently of any other of the embodiments or in any feasible combination with one or more others of the embodiments.

**EP 4 650 987 A1**

**Claims**

1. A computer-implemented method comprising:

categorizing each datapoint in a training dataset into one of a plurality of primary subsets based on values of the datapoint in respect of at least first and second attributes so that each primary subset corresponds to specific values of at least the first and second attributes, wherein each datapoint is defined by a plurality of attributes including the at least first and second attributes;

selecting a specific value in respect of the first attribute and dividing each of the primary subsets corresponding to the selected value of the first attribute into a plurality of auxiliary subsets;

for each of the primary subsets corresponding to the selected value of the first attribute, downsampling the plurality of auxiliary subsets with respect to the other primary subsets, respectively, to generate a plurality of downsampled auxiliary subsets; and

generating a downsampled training dataset for training a machine learning, ML, model to predict the first attribute, the downsampled training dataset comprising the datapoints of the downsampled auxiliary subsets and datapoints of the primary subsets other than the primary subsets corresponding to the selected value of the first attribute,

wherein the downsampling comprises:

for each datapoint in the auxiliary subset concerned, computing an average distance to the k furthest datapoints of the primary subset concerned in respect of the plurality of attributes other than the at least first and second attributes; and

removing n datapoints of the auxiliary subset concerned having the largest computed average distance to generate the downsampled auxiliary subset concerned, where k and n are positive integers.

2. The computer-implemented method as claimed in claim 1, wherein selecting the specific value in respect of the first attribute comprises, when the training dataset is imbalanced in respect of the first attribute, selecting the most common value among the training dataset in respect of the first attribute.

3. The computer-implemented method as claimed in claim 1 or claim 2, wherein dividing each of the primary subsets corresponding to the selected value in respect of the first attribute into a plurality of auxiliary subsets comprises, for each primary subset corresponding to the selected value in respect of the first attribute, dividing the primary subset into a number of auxiliary subsets equal to the number of other primary subsets.

4. The computer-implemented method as claimed in any of the preceding claims, wherein dividing each of the primary subsets corresponding to the selected value in respect of the first attribute into a plurality of auxiliary subsets comprises, for each primary subset corresponding to the selected value in respect of the first attribute, dividing the primary subset into the plurality of auxiliary subsets sized proportionally to the other primary subsets, respectively.

5. The computer-implemented method as claimed in any of claims 1 to 3, wherein, for each of the primary subsets corresponding to the selected value in respect of the first attribute, the corresponding auxiliary subsets comprise numbers of datapoints proportional to the other primary subsets, respectively.

6. The computer-implemented method as claimed in any of the preceding claims, wherein the computer-implemented method further comprises:

computing a fairness measure of the downsampled training dataset;

if the fairness measure fails to meet a threshold fairness, for each of the primary subsets corresponding to the selected value of the first attribute further downsampling the plurality of auxiliary subsets with respect to the other primary subsets, respectively, to generate a plurality of further downsampled auxiliary subsets; and

generating a further downsampled training dataset for training the ML model to predict the first attribute, the downsampled training dataset comprising the datapoints of the further downsampled auxiliary subsets and datapoints of the primary subsets other than the primary subsets corresponding to the selected value of the first attribute.

7. The computer-implemented method as claimed in any of the preceding claims, wherein the computer-implemented method further comprises:

after downsampling, computing a ratio between the largest of the primary subsets and the smallest of the primary subsets;

when the ratio fails to meet a threshold ratio, for each of the primary subsets corresponding to the selected value of the first attribute further downsampling the plurality of auxiliary subsets with respect to the other primary subsets, respectively, to generate a plurality of further downsampled auxiliary subsets; and

generating a further downsampled training dataset for training the ML model to predict the first attribute, the further downsampled training dataset comprising the datapoints of the further downsampled auxiliary subsets and datapoints of the primary subsets other than the primary subsets corresponding to the selected value of the first attribute.

8. The computer-implemented method as claimed in any of the preceding claims, wherein the training dataset comprises medical data and wherein each datapoint of the training dataset relates to a human subject or patient.

9. The computer-implemented method as claimed in any of the preceding claims, wherein the first attribute is the presence of a disease or condition.

10. The computer-implemented method as claimed in any of the preceding claims, further comprising training the ML model using the downsampled training dataset.

11. The computer-implemented method as claimed in claim 10, further comprising using the ML model to predict the first attribute in respect of a new data instance.

12. The computer-implemented method as claimed in claim 10 or 11, further comprising using the ML model to predict the presence or absence of a disease or condition.

13. The computer-implemented method as claimed in any of claims 1 to 7, wherein the training dataset comprises medical data and wherein each datapoint of the training dataset relates to a human subject or patient, wherein the first attribute is the presence of a disease or condition, wherein the computer-implemented method further comprises training the ML model using the downsampled training dataset and using the ML model to predict the first attribute in respect of a new human subject or patient, and wherein the computer-implemented method further comprises outputting a diagnosis in respect of the new human subject or patient comprising the prediction of the presence or absence of the disease or condition.

14. A computer program which, when run on a computer, causes the computer to carry out a method comprising:

categorizing each datapoint in a training dataset into one of a plurality of primary subsets based on values of the datapoint in respect of at least first and second attributes so that each primary subset corresponds to specific values of at least the first and second attributes, wherein each datapoint is defined by a plurality of attributes including the at least first and second attributes;

selecting a specific value in respect of the first attribute and dividing each of the primary subsets corresponding to the selected value of the first attribute into a plurality of auxiliary subsets;

for each of the primary subsets corresponding to the selected value of the first attribute, downsampling the plurality of auxiliary subsets with respect to the other primary subsets, respectively, to generate a plurality of downsampled auxiliary subsets; and

generating a downsampled training dataset for training a machine learning, ML, model to predict the first attribute, the downsampled training dataset comprising the datapoints of the downsampled auxiliary subsets and the datapoints of the primary subsets other than the primary subsets corresponding to the selected value of the first attribute,

wherein the downsampling comprises:

for each datapoint in the auxiliary subset concerned, computing an average distance to the k furthest datapoints of the primary subset concerned in respect of the plurality of attributes other than the at least first and second attributes; and

removing n datapoints of the auxiliary subset concerned having the largest computed average distance to generate the downsampled auxiliary subset concerned, where k and n are positive integers.

15. An information processing apparatus comprising a memory and a processor connected to the memory, wherein the processor is configured to:

categorize each datapoint in a training dataset into one of a plurality of primary subsets based on values of the datapoint in respect of at least first and second attributes so that each primary subset corresponds to specific values of at least the first and second attributes, wherein each datapoint is defined by a plurality of attributes including the at least first and second attributes;

select a specific value in respect of the first attribute and divide each of the primary subsets corresponding to the selected value of the first attribute into a plurality of auxiliary subsets;

for each of the primary subsets corresponding to the selected value of the first attribute, downsample the plurality of auxiliary subsets with respect to the other primary subsets, respectively, to generate a plurality of downsampled auxiliary subsets; and

generate a downsampled training dataset for training a machine learning, ML, model to predict the first attribute, the downsampled training dataset comprising the datapoints of the downsampled auxiliary subsets and the datapoints of the primary subsets other than the primary subsets corresponding to the selected value of the first attribute,

wherein the downsampling comprises:

for each datapoint in the auxiliary subset concerned, computing an average distance to the k furthest datapoints of the primary subset concerned in respect of the plurality of attributes other than the at least first and second attributes; and

removing n datapoints of the auxiliary subset concerned having the largest computed average distance to generate the downsampled auxiliary subset concerned, where k and n are positive integers.

**Amended claims in accordance with Rule 137(2) EPC.**

1. A computer-implemented method comprising:

   categorizing (S10) each datapoint in a training dataset into one of a plurality of primary subsets based on values of the datapoint in respect of at least first and second attributes so that each primary subset corresponds to specific values of at least the first and second attributes, wherein each datapoint is defined by a plurality of attributes including the at least first and second attributes;

   selecting (S20) a specific value in respect of the first attribute and dividing (S30) each of the primary subsets corresponding to the selected value of the first attribute into a plurality of auxiliary subsets;

   for each of the primary subsets corresponding to the selected value of the first attribute, downsampling (S40) the plurality of auxiliary subsets with respect to the other primary subsets, respectively, to generate a plurality of downsampled auxiliary subsets; and

   generating (S50) a downsampled training dataset for training a machine learning, ML, model to predict the first attribute, the downsampled training dataset comprising the datapoints of the downsampled auxiliary subsets and datapoints of the primary subsets other than the primary subsets corresponding to the selected value of the first attribute,

   wherein the downsampling comprises:

   for each datapoint in the auxiliary subset concerned, computing (S42) an average distance to the k furthest datapoints of the primary subset concerned in respect of the plurality of attributes other than the at least first and second attributes; and

   removing (S43) n datapoints of the auxiliary subset concerned having the largest computed average distance to generate the downsampled auxiliary subset concerned, where k and n are positive integers,

   wherein the computer-implemented method comprises using, in parallel, a plurality of processors with different sizes for downsampling the auxiliary datasets, wherein the downsampling of the auxiliary datasets is assigned to the plurality of processors according to the sizes of the auxiliary datasets so that larger auxiliary datasets are downsampled using larger-sized processors.

2. The computer-implemented method as claimed in claim 1, wherein selecting (S20) the specific value in respect of the first attribute comprises, when the training dataset is imbalanced in respect of the first attribute, selecting the most common value among the training dataset in respect of the first attribute.

3. The computer-implemented method as claimed in claim 1 or claim 2, wherein dividing (S30) each of the primary subsets corresponding to the selected value in respect of the first attribute into a plurality of auxiliary subsets comprises, for each primary subset corresponding to the selected value in respect of the first attribute, dividing the primary subset into a number of auxiliary subsets equal to the number of other primary subsets.

4.  The computer-implemented method as claimed in any of the preceding claims, wherein dividing (S30) each of the primary subsets corresponding to the selected value in respect of the first attribute into a plurality of auxiliary subsets comprises, for each primary subset corresponding to the selected value in respect of the first attribute, dividing the primary subset into the plurality of auxiliary subsets sized proportionally to the other primary subsets, respectively.

5.  The computer-implemented method as claimed in any of claims 1 to 3, wherein, for each of the primary subsets corresponding to the selected value in respect of the first attribute, the corresponding auxiliary subsets comprise numbers of datapoints proportional to the other primary subsets, respectively.

6.  The computer-implemented method as claimed in any of the preceding claims, wherein the computer-implemented method further comprises:

    computing a fairness measure of the downsampled training dataset;
    if the fairness measure fails to meet a threshold fairness, for each of the primary subsets corresponding to the selected value of the first attribute further downsampling the plurality of auxiliary subsets with respect to the other primary subsets, respectively, to generate a plurality of further downsampled auxiliary subsets; and
    generating a further downsampled training dataset for training the ML model to predict the first attribute, the downsampled training dataset comprising the datapoints of the further downsampled auxiliary subsets and datapoints of the primary subsets other than the primary subsets corresponding to the selected value of the first attribute.

7.  The computer-implemented method as claimed in any of the preceding claims, wherein the computer-implemented method further comprises:

    after downsampling, computing a ratio between the largest of the primary subsets and the smallest of the primary subsets;
    when the ratio fails to meet a threshold ratio, for each of the primary subsets corresponding to the selected value of the first attribute further downsampling the plurality of auxiliary subsets with respect to the other primary subsets, respectively, to generate a plurality of further downsampled auxiliary subsets; and
    generating a further downsampled training dataset for training the ML model to predict the first attribute, the further downsampled training dataset comprising the datapoints of the further downsampled auxiliary subsets and datapoints of the primary subsets other than the primary subsets corresponding to the selected value of the first attribute.

8.  The computer-implemented method as claimed in any of the preceding claims, wherein the training dataset comprises medical data and wherein each datapoint of the training dataset relates to a human subject or patient.

9.  The computer-implemented method as claimed in any of the preceding claims, wherein the first attribute is the presence of a disease or condition.

10. The computer-implemented method as claimed in any of the preceding claims, further comprising training the ML model using the downsampled training dataset.

11. The computer-implemented method as claimed in claim 10, further comprising using the ML model to predict the first attribute in respect of a new data instance.

12. The computer-implemented method as claimed in claim 10 or 11, further comprising using the ML model to predict the presence or absence of a disease or condition.

13. The computer-implemented method as claimed in any of claims 1 to 7, wherein the training dataset comprises medical data and wherein each datapoint of the training dataset relates to a human subject or patient, wherein the first attribute is the presence of a disease or condition, wherein the computer-implemented method further comprises training the ML model using the downsampled training dataset and using the ML model to predict the first attribute in respect of a new human subject or patient, and wherein the computer-implemented method further comprises outputting a diagnosis in respect of the new human subject or patient comprising the prediction of the presence or absence of the disease or condition.

14. A computer program which, when run on a computer, causes the computer to carry out a method comprising:

categorizing (S10) each datapoint in a training dataset into one of a plurality of primary subsets based on values of the datapoint in respect of at least first and second attributes so that each primary subset corresponds to specific values of at least the first and second attributes, wherein each datapoint is defined by a plurality of attributes including the at least first and second attributes;

selecting (S20) a specific value in respect of the first attribute and dividing (S30) each of the primary subsets corresponding to the selected value of the first attribute into a plurality of auxiliary subsets;

for each of the primary subsets corresponding to the selected value of the first attribute, downsampling (S40) the plurality of auxiliary subsets with respect to the other primary subsets, respectively, to generate a plurality of downsampled auxiliary subsets; and

generating (S50) a downsampled training dataset for training a machine learning, ML, model to predict the first attribute, the downsampled training dataset comprising the datapoints of the downsampled auxiliary subsets and the datapoints of the primary subsets other than the primary subsets corresponding to the selected value of the first attribute,

wherein the downsampling comprises:

for each datapoint in the auxiliary subset concerned, computing (S42) an average distance to the k furthest datapoints of the primary subset concerned in respect of the plurality of attributes other than the at least first and second attributes; and

removing (S43) n datapoints of the auxiliary subset concerned having the largest computed average distance to generate the downsampled auxiliary subset concerned, where k and n are positive integers,

wherein the method comprises using, in parallel, a plurality of processors with different sizes for downsampling the auxiliary datasets, wherein the downsampling of the auxiliary datasets is assigned to the plurality of processors according to the sizes of the auxiliary datasets so that larger auxiliary datasets are downsampled using larger-sized processors.

15. An information processing apparatus (10) comprising a memory (994) and a processor (993) connected to the memory (994), wherein the processor (993) is configured to:

categorize each datapoint in a training dataset into one of a plurality of primary subsets based on values of the datapoint in respect of at least first and second attributes so that each primary subset corresponds to specific values of at least the first and second attributes, wherein each datapoint is defined by a plurality of attributes including the at least first and second attributes;

select a specific value in respect of the first attribute and divide each of the primary subsets corresponding to the selected value of the first attribute into a plurality of auxiliary subsets;

for each of the primary subsets corresponding to the selected value of the first attribute, downsample the plurality of auxiliary subsets with respect to the other primary subsets, respectively, to generate a plurality of downsampled auxiliary subsets; and

generate a downsampled training dataset for training a machine learning, ML, model to predict the first attribute, the downsampled training dataset comprising the datapoints of the downsampled auxiliary subsets and the datapoints of the primary subsets other than the primary subsets corresponding to the selected value of the first attribute,

wherein the downsampling comprises:

for each datapoint in the auxiliary subset concerned, computing an average distance to the k furthest datapoints of the primary subset concerned in respect of the plurality of attributes other than the at least first and second attributes; and

removing n datapoints of the auxiliary subset concerned having the largest computed average distance to generate the downsampled auxiliary subset concerned, where k and n are positive integers,

wherein the processor (993) comprises a plurality of secondary processors with different sizes for, in parallel, downsampling the auxiliary datasets, wherein the downsampling of the auxiliary datasets is assigned to the plurality of secondary processors according to the sizes of the auxiliary datasets so that larger auxiliary datasets are downsampled using larger-sized secondary processors.

FIG. 1

FIG. 2

EP 4 650 987 A1

FIG. 3

S110     S130     S140     S150

| Data | → | Training set (S110) | → | Fair downsampling (S130) | → | Train classifier (S140) |
|---|---|---|---|---|---|---|

Testing set (S120)

Evaluation (S150)

FIG. 4

Split dataset $D$ into groups $G_i$ and classes $C_i$

<u>S210</u>

Identify majority and minority classes, $M_i$ and $m_i$, for protected group $i$

<u>S220</u>

Split $M_1$ into 3 random non-overlapping sets $S_i$ with size that can be proportional to $m_1$, $m_2$, $M_2$

<u>S231</u>

Split $M_2$ into 3 random sets $T_i$

<u>S232</u>

<u>S241</u> Downsample the pairs $(S_1, m_1)$, $(S_2, m_2)$, $(S_3, M_2)$

<u>S242</u> Downsample the pairs $(T_1, m_1)$, $(T_2, m_2)$, $(T_3, M_1)$

FIG. 5

repeat for all $i$

S310 — Pair $(S, m)$ to be downsampled

S320 — Select $s_i \in S$

S330 — Using KNN calculate average distance $d_i$, of $s_i$ with the $n = 3$ furthest samples of $m$

S340 — Form the set of all distances $d_i$

S350 — Keep samples in $S$ with the smallest $d_i$

FIG. 6

FIG. 7

FIG. 8

FIG. 9

FIG. 10

FIG. 11

Input data $D = \{X, Y, S\}$

**S1** Split input data into clusters $C_{y,s}$ of groups and classes

**S2** Calculation of size of each cluster $C_{y,s}$

**S3**
$$M = \max_{y,s}|C_{y,s}|$$
$$m = \min_{y,s}|C_{y,s}|$$

$\frac{m}{M} \geq p$ → END

$\frac{m}{M} < p$

**S4** Selection of cluster $C_j$ to be downsampled

**S5** Split of $C_j$ into $\bigcup_k C_j^k = C_j$ using random sampling

**S6** Selection of cluster $C_j^i \neq C_j$

repeat for all i

**S7** Downsampling $C_j^i$, $k = 1$ with respect to $C_j$

repeat for all j with different k

**S8** compute fairness $f$

$f < q$ → **S9** increase downsampling

$f \geq q$

$p$: hyperparameter amount of downsampling ($p = 1$ balance)
$q$: desired amount of fairness

S10 — Categorize training dataset into plurality of primary subsets

S20 — Select a value of first attribute

S30 — Divide each primary subset corresponding to selected value into auxiliary subsets

S40 — For each primary subset corresponding to the selected value, downsample the auxiliary subsets with respect to the other primary subsets, respectively, to generate downsampled auxiliary subsets

S50 — Generate downsampled training dataset

FIG. 12

EP 4 650 987 A1

S41 — Select auxiliary subset

S42 — For each datapoint in auxiliary subset, compute average distance to k furthest datapoints of corresponding primary subset

S43 — Remove n datapoints having the largest average distance

S45 — Auxiliary subset remaining which has not been downsampled?

yes

no

S46 — End

FIG. 13

FIG. 14

10

PROCESSOR

993

MEMORY

994

992

995

DISPLAY

996

INPUT

997

NETWORK/F

FIG. 15

Europäisches
Patentamt

European
Patent Office

Office européen
des brevets

# EUROPEAN SEARCH REPORT

**Application Number**

EP 24 38 6053

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SALAZAR TERESA ET AL:  "FAWOS: Fairness-Aware Oversampling Algorithm Based on Distributions of Sensitive Attributes", IEEE ACCESS, IEEE, USA, vol. 9, 27 May 2021 (2021-05-27), pages 81370-81379, XP011859624, DOI: 10.1109/ACCESS.2021.3084121 [retrieved on 2021-06-08] * page 81372 * | 1-15 | INV. G06F16/906 G06N20/00 |
| | ----- | | |
| | | | **TECHNICAL FIELDS SEARCHED (IPC)** |
| | | | G06F G06N |

The present search report has been drawn up for all claims

| Place of search | Date of completion of the search | Examiner |
|---|---|---|
| The Hague | 18 October 2024 | Rameseder, Jonathan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    ..........................................................................................
& : member of the same patent family, corresponding
    document

EPO FORM 1503 03.82 (P4C01)